# EUROPEAN PATENT APPLICATION

(11) **EP 2 306 549 A1**
(43) Date of publication of application: **06.04.2011**
(21) Application number: 09809595.3
(22) Date of filing: 28.08.2009
(51) Int. Cl.: H01M 2/10

(54) **BATTERY PACK AND ELECTRONIC DEVICE CONFIGURATIONS PROVIDED WITH SAID BATTERY PACKS**

(30) Priority: 29.08.2008 JP 2008221377
(71) Applicant: Panasonic Corporation, Kadoma-shi Osaka 571-8501 (JP)
(72) Inventor: UEDA, Tomohiro, 1-61 Shiromi 2-chome, Chuo-ku, Osaka-shi, Osaka 540-6207 (JP)
(74) Representative: TBK-Patent
(86) International application number: PCT/JP2009/004212
(87) International publication number: WO 2010/023940

(57) **Abstract**

This invention relates to an improvement in battery packs. The invention intends to provide a highly flexible battery pack and an electronic device assembly using the same. The battery pack of the invention includes an elastic sheet and at least one coin battery embedded in the elastic sheet. In a preferable embodiment of the invention, the electronic device assembly includes a laminate of the battery pack and a flexible electronic device. The electronic device is driven by power supplied through positive and negative terminals of the battery pack. In another preferable embodiment of the invention, the electronic device assembly includes the battery pack and a device embedded in the elastic sheet of the battery pack, and the device is driven by power supplied by the at least one coin battery.

## Description

### [Technical Field]

This invention relates to a flexible battery pack comprising one or more coin batteries embedded in an elastic sheet.

### [Background Art]

Small coin batteries have been widely used as the driving power source for miniaturized electronic devices. In recently years, electronic devices are increasingly becoming smaller, thinner, and lighter. In the field of miniaturized electronic devices, there is demand for driving power sources which are smaller and thinner.

Examples of miniaturized electronic devices include IC cards containing IC chips. IC cards are widely used in such fields as entrance-exit management, automatic ticket gates, and information-communications. The use of thin batteries as the driving power source for such IC cards is being examined. A thin battery comprises a thin electrode assembly contained in a thin housing. Such an electrode assembly includes a thin positive electrode layer, a thin electrolyte layer, and a thin negative electrode layer. Also, the housing is formed of a thin metal film or a thin laminate film comprising a synthetic resin layer and a metal layer.

In the medical field, wearable portable devices have recently been developed in order to allow doctors and the like to monitor biological information of patients and the like. Wearable portable devices are wom directly on the body all day to constantly measure biological information, such as blood pressure, body temperature, or pulse, and transmit the measured information by radio. Since such a wearable portable device is tightly fitted to a living body while in use, the wearable portable device is required to be flexible enough to cause no discomfort even when it is tightly fitted for a long time. Therefore, the driving power source for such a wearable portable device is also required to be highly flexible. The use of flexible thin batteries as the power source for wearable portable devices is also being examined.

Specifically, for example, PTL 1 discloses a thin secondary battery comprising: a power generating element including a positive electrode, a highly flexible polymer electrolyte layer, and a negative electrode; and a housing containing the power generating element, the housing being made of a laminate film which uses an aluminum foil as a core material. It discloses that at least one of the positive and negative electrodes of the thin secondary battery is a sheet electrode which comprises a current collector composed mainly of carbon fibers and an electrode material mixture supported on the current collector.

Also, PTL 2 discloses a thin, flexible lithium battery which includes a positive electrode layer, a solid electrolyte layer, and a negative electrode layer which are supported on a substrate. The thin lithium battery can be produced by forming a positive electrode layer, a solid electrolyte layer, and a negative electrode layer on a surface of a resin substrate by vapor deposition or coating.

### [Citation List]

### [Patent Literatures]

PTL 1: Japanese Laid-Open Patent Publication No. 2002-63938
PTL 2: Japanese Laid-Open Patent Publication No. 2008-171599

### [Summary of Invention]

### [Technical Problem]

Thin batteries as disclosed in PTL 1 and PTL 2 need to have a large area in the direction perpendicular to the thickness direction in order to secure sufficient electrical capacity. They also need to have a uniform and sufficient adhesion between the positive electrode layer, the electrolyte layer, and the negative electrode layer in the thickness direction over the large area. In order to retain such an adhesion between the respective layers over the large area, the power generating element including the positive electrode layer, the electrolyte layer, and the negative electrode layer is sealed in the housing, and the pressure inside the housing is reduced to decrease gaps inside the battery. However, thin batteries produced by such a method become hard and inflexible, since they are sealed under a reduced pressure and there are no gaps inside the batteries. When such an inflexible thin battery is used for a wearable portable device, if it is wom for a long time, it gives a poor fit due to the rigidity of the battery itself. Further, when the battery is partially bent or deformed for a long time, the bent or deformed portion is intensively subjected to a stress, which may result in deterioration of battery performance.

Also, in the method as disclosed in PTL 1, in which the battery element is sealed in a housing made of a metal film or a laminate film, the open edge of the housing is thermally welded. Thus, a polymer electrolyte with low heat resistance cannot be used. Further, in the method as disclosed in PTL 2, in which very thin electrode and electrolyte layers are formed by a method such as vapor deposition, the productivity of thin batteries is low since a gas phase process is necessary.

These problems are solved by the invention, and an object of the invention is to provide a highly flexible battery pack with high productivity.

### [Solution to Problem]

The battery pack in one aspect of the invention includes an elastic sheet and at least one coin battery embedded in the elastic sheet.

Also, the electronic device assembly in another aspect of the invention includes a laminate of the battery pack and a flexible electronic device, wherein the electronic device is driven by power supplied through positive and negative terminals of the battery pack.

### [Advantageous Effects of Invention]

According to the invention, since the coin battery or batteries are embedded in the elastic sheet, the battery pack itself has high flexibility.
The objects, features, aspects, and advantages of the invention will become more apparent from the following detailed description.

### [Brief Description of Drawings]

FIG. 1 is a schematic top view of a battery pack according to an embodiment of the invention;
FIG. 2 is a schematic longitudinal sectional view of the battery pack of FIG. 1 taken along the line II-II;
FIG. 3 is a schematic longitudinal sectional view of an exemplary electronic device assembly comprising the battery pack of FIG. 1 and an external device connected to the pack;
FIG. 4 is a schematic top view of a battery pack according to another embodiment of the invention;
FIG. 5 is a schematic longitudinal sectional view of the battery pack of FIG. 4 taken along the line V-V;
FIG. 6 is a schematic top view of a battery pack according to still another embodiment of the invention;
FIG. 7 is a schematic longitudinal sectional view of the battery pack of FIG. 6 taken along the line VII-VII;
FIG. 8 is a schematic top view of a battery pack according to still another embodiment of the invention;
FIG. 9 is a schematic longitudinal sectional view of the battery pack of FIG. 8 taken along the line IX-IX;
FIG. 10 is a schematic top view of a battery pack according to still another embodiment of the invention;
FIG. 11 is a schematic longitudinal sectional view of the battery pack of FIG. 10 taken along the line XI-XI;
FIG. 12 is a schematic top view of a battery pack according to still another embodiment of the invention;
FIG. 13 is a schematic longitudinal sectional view of the battery pack of FIG. 12 taken along the line XIII-XIII;
FIG. 14 is a schematic top view of a battery pack according to still another embodiment of the invention;
FIG. 15 is a schematic longitudinal sectional view of the battery pack of FIG. 14 taken along the line XV-XV;
FIG. 16 is a schematic top view of a battery pack according to still another embodiment of the invention;
FIG. 17 is a schematic longitudinal sectional view of the battery pack of FIG. 16 taken along the line XVII-XVII;
FIG. 18 is a schematic top view of a battery pack according to still another embodiment of the invention;
FIG. 19 is a schematic longitudinal sectional view of the battery pack of FIG. 18 taken along the line XIX-XIX;
FIG. 20 is a schematic longitudinal sectional view of an electronic device assembly according to another embodiment of the invention;
FIG. 21 is a schematic view of the electronic device assembly of FIG. 20 which is tightly fitted to a living body;
FIG. 22 is schematic sectional views showing an exemplary method for producing the battery pack of FIG. 1;
   and
FIG. 23 is a schematic longitudinal sectional view of a battery pack according to still another embodiment of the invention.

### [Description of Embodiments]

### [First Embodiment]

The battery pack of this embodiment includes an elastic sheet and at least one coin battery embedded in the elastic sheet. The positive electrode of the at least one coin battery or the positive terminal connected to the positive electrode and the negative electrode or the negative terminal connected to the negative electrode are exposed at a surface of the elastic sheet.

This embodiment is hereinafter described with reference to drawings.

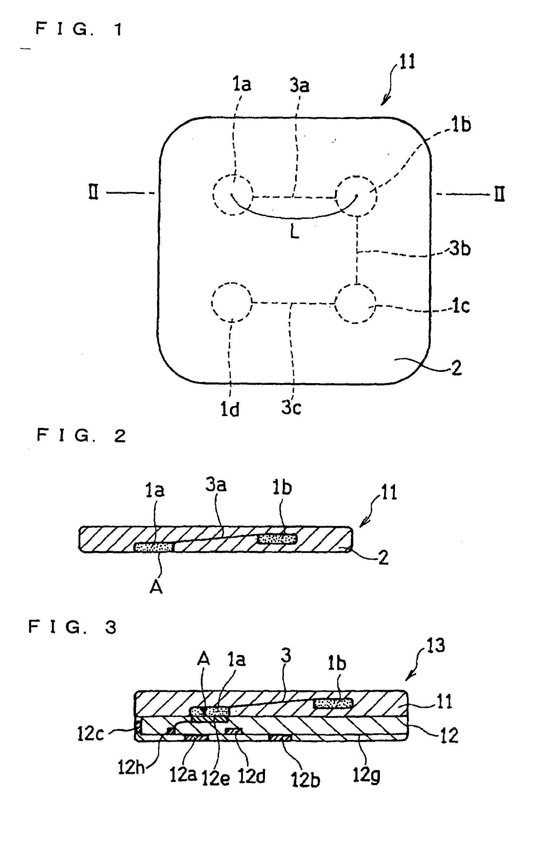

FIG. 1 is a top view of a battery pack according to this embodiment, and FIG. 2 is a longitudinal sectional view of the battery pack of FIG. 1 taken along the line II-II. In the top view of FIG. 1, coin batteries 1 (1a to 1d) and wiring 3 (3a, 3b, and 3c) embedded in the battery pack 11 are shown by the dotted lines.

The battery pack 11 of FIG. 1 includes a square elastic sheet 2 and four coin batteries 1a to 1d embedded in the elastic sheet 2.
The coin batteries 1 are shaped like coins, and the upper face of the coin-shaped housing is the positive electrode, while the lower face thereof is the negative electrode.

In the battery pack 11 of FIG. 1, the coin batteries 1a to 1d are connected by the wiring 3 (3a, 3b, and 3c). Specifically, the positive electrode of the coin battery 1a is connected to the negative electrode of the coin battery 1b, the positive electrode of the coin battery 1b is connected to the negative electrode of the coin battery 1c, and the positive electrode of the coin battery 1c is connected to the negative electrode of the coin battery 1d. The negative electrode A of the coin battery 1a or the negative terminal connected to the negative electrode A is exposed at a surface of the elastic sheet 2, as illustrated in FIG. 2. Also, the positive electrode B of the coin battery 1d or the positive terminal connected to the positive electrode B is exposed at the surface of the elastic sheet 2, although not shown in FIG. 2.

In the battery pack 1, the coin batteries 1a to 1d are connected in series, but the coin batteries 1a to 1d may be connected in parallel. When the coin batteries 1 are connected in series, a high voltage discharge becomes possible. Also, when the coin batteries 1 are connected in parallel, a high load discharge becomes possible. By tuming the coin batteries upside down, the orientation of the positive and negative electrodes can be freely changed. The number of the coin batteries 1 embedded in the elastic sheet 2 may be only one.

The coin batteries 1 are embedded in the elastic sheet 2. Thus, the battery pack 11 has such flexibility that it is capable of easily deforming according to external stress.
Examples of materials for the elastic sheet 2 include: rubber materials such as silicone rubber, fluorosilicone rubber, and fluorocarbon rubber; thermoplastic olefin elastomers, plastic polyurethane elastomers, and thermoplastic elastomers such as styrene-butadiene block polymer elastomers, styrene-isoprene block polymer elastomers, and styrene-ethylene-butylene block polymer elastomers; polyolefins such as polyethylene; polyurethane; ternary copolymers comprising ethylene-propylene-diene monomers; and polyamides. Among them, silicone rubber is preferable since it has good durability such as weather resistance, oil resistance, and chemical resistance. The elastic sheet 2 may contain other resin components, a plasticizer, an inorganic filler, etc., if necessary.
The modulus of elasticity of the elastic sheet 2 is preferably 0.1 to 50 MPa, and more preferably about 1 to 10 MPa.

Examples of the coin batteries 1 include lithium batteries, lithium ion batteries, lithium polymer batteries, manganese dry batteries, alkaline dry batteries, air batteries, nickel cadmium batteries, nickel-metal hydride batteries, and portable fuel cells. Also, the coin batteries 1 may be either primary batteries or secondary batteries. Lithium coin batteries are particularly preferable since they can produce high electromotive force stably while having a small diameter and good productivity.

The diameter of the coin batteries is preferably 20 mm or less, more preferably 12 mm or less, and even more preferably 10 mm or less, since the flexibility of the battery pack 11 is not impaired.

While the distance L between the adjacent coin batteries 1 is not particularly limited, it is preferably about 5 to 200 mm, and more preferably about 10 to 100 mm. When the distance L is too short, if one or both of the two coin batteries 1 are positioned near slack, the slack may be reduced. Also, when the distance L is too long, if one or both of the two coin batteries 1 are positioned near slack, the wiring 3 may not deform sufficiently together with deformation of the battery pack 11 and may be damaged.

While the thickness of the battery pack 11 is not particularly limited, it is preferably, for example, 0.5 to 20 mm, and more preferably about 1.0 to 10 mm. If the battery pack 11 is too thin, it may not sufficiently protect the coin batteries 1. If the battery pack 11 is too thick, its flexibility tends to lower.

In the battery pack 11, the coin batteries 1 electrically connected by the wiring 3 are embedded in the elastic sheet 2. The materials for the wiring 3 include, but are not particularly limited to, conventionally known conductive materials such as copper, copper alloys, carbon, platinum, gold, silver, titanium, nickel, aluminum, and iron.

The wiring 3 can be formed by, for example, connecting the electrodes of the coin batteries 1 with electric wires, or forming a thin-film circuit on a surface of a predetermined flexible substrate by vapor deposition or plating. In forming the wiring, it is preferable to dispose the coin batteries 1 in the horizontal direction of the elastic sheet 2 and dispose wiring mainly in the central part of the thickness of the elastic sheet 2 to electrically connect them. For example, when the elastic sheet 2 is folded so that the crease is concave downward (mountain fold), the surface side stretches while the back side shrinks. Thus, when wiring is disposed on the surface side of the elastic sheet 2, if the elastic sheet 2 deforms greatly, the wiring is stretched and may be damaged. On the other hand, when wiring is disposed in the central part of the thickness of the elastic sheet, the wiring is less likely to be stretched due to small shrinkage.

In the battery pack 11, the electrodes of the coin batteries 1 or electrode terminals connected to the electrodes are exposed at the surface of the elastic sheet 2. The exposed electrodes or electrode terminals connected to the electrodes are to be connected to the electrode terminals of an electronic device to supply electric power.

FIG. 2 is a longitudinal sectional view of the battery pack 11 of FIG. 1 taken along the line II-II. As illustrated in FIG. 2, the negative electrode A of the coin battery 1a is exposed at a surface of the battery pack 11. Also, although not illustrated in FIG. 2, the positive electrode B of the coin battery 1d is exposed at the surface of the battery pack 11.

The negative electrode A of the coin battery 1a and the positive electrode B of the coin battery 1d which are exposed at the surface of the battery pack 11 serve as terminals for supplying power to an electronic device. By connecting the negative electrode A and the positive electrode B exposed at the surface of the battery pack 11 to the electrode terminals of the electronic device, power can be supplied to the electronic device.

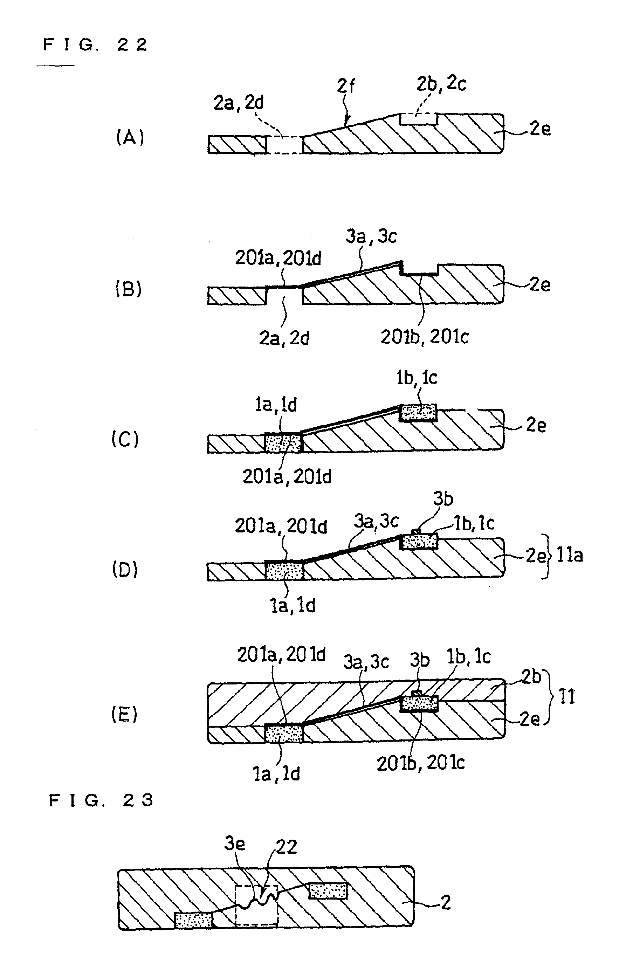

Next, an exemplary method for producing the battery pack 11 is described. FIG. 22 (A) to (E) are schematic sectional views showing the steps for producing the battery pack 11.

First, an elastic sheet unit 2e having a shape as illustrated in FIG. 22 (A) is prepared. The elastic sheet unit 2e has through-holes 2a and 2d for receiving the coin battery 1a and the coin battery 1d, respectively, recesses 2b and 2c for receiving the coin battery 1b and the coin battery 1c, respectively, and a slope 2f. The elastic sheet unit 2e having such a shape can be produced by injection molding using a thermoplastic elastomer, or by injecting a liquid elastomer into a predetermined mold and allowing it to set.

Next, as illustrated in FIG. 22 (B), electrode terminals 201a and 201b connected by the wiring 3a are inserted into the through-hole 2a and the recess 2b, respectively. Likewise, electrode terminals 201c and 201d connected by the wiring 3c are inserted into the recess 2c and the through-hole 2d, respectively.

Subsequent, as illustrated in FIG. 22 (C), the coin battery 1a is fitted so that its positive electrode is in contact with the electrode terminal 201a while its negative electrode is exposed at the outer surface of the elastic sheet unit 2e through the through-hole 2a. Also, the coin battery 1b is fitted so that its negative electrode is in contact with the electrode terminal 201b. Also, the coin battery 1c is fitted so that its negative electrode is in contact with the electrode terminal 201c in the recess 2c. Also, the coin battery 1d is fitted so that its positive electrode is in contact with the electrode terminal 201d while its positive electrode is exposed at the outer surface of the elastic sheet unit 2e through the through-hole 2d. Further, the negative electrode of the coin battery 1b is connected to the positive electrode of the coin battery 1c by the wiring 3b. In this manner, an assembly 11a including the coin batteries 1a to 1d connected in series, as illustrated in FIG. 22 (D), can be obtained.

Thereafter, as illustrated in FIG. 22 (E), the assembly 11a is sealed with an elastic sheet unit 2b. It can be sealed with the elastic sheet unit 2b by, for example, placing the assembly 11a into a predetermined mold, injecting a liquid elastomer into the mold, and allowing it to set, or by injection insert molding using a thermoplastic elastomer. By such a method, the battery pack 11 as illustrated in FIG. 1 and FIG. 2 can be obtained.

As illustrated in FIG. 23, in the elastic sheet 2, the wiring 3 (3a, 3b, and 3c) preferably has slack 3e so that it is longer than the shortest distance between the electrodes of the coin batteries. Since there is the slack 3e, when the battery pack 11 becomes deformed, damage of the wiring 3 is suppressed. It is preferable to dispose the slack 3e in a void 22 that is formed in the elastic sheet 2 at a given position along the wiring 3, in order to facilitate the movement of the slack.

### [Second Embodiment]

This embodiment describes an exemplary electronic device assembly in which the battery pack 11 is used as the driving power source for a flexible electronic device.
FIG. 3 illustrates the structure of a wearable portable device 13 which constantly measures biological information such as blood pressure, body temperature, or pulse and transmits the measured information by radio.

A biological information measuring device 12 includes a temperature sensor 12a, a pressure sensor 12b, an information transmitting unit 12c, a control unit 12d with a predetermined control circuit, a negative terminal 12e, and a positive terminal (not shown).

In the wearable portable device 13, the battery pack 11 is used as the driving power source for the biological information measuring device 12.
Specifically, for example, as illustrated in FIG. 3, the negative terminal 12e exposed at a surface of the biological information measuring device 12 is connected to the negative electrode A of the coin battery 1a embedded in the battery pack 11. Likewise, the positive terminal exposed at the surface of the biological information measuring device 12 is connected to the positive electrode of the coin battery 1d embedded in the battery pack 11. In this manner, power can be supplied to the biological information measuring device 12 from the battery pack 11.

The biological information measuring device 12 comprises a circuit board covered with a deformable material, and the circuit board includes electronic components such as the temperature sensor 12a, the pressure sensor 12b, the information transmitting unit 12c, and the control unit 12d which are mounted on the surface of a flexible printed circuit board 12g. A negative power supply portion 12h and a positive power supply portion (not shown) formed on the surface of the flexible printed circuit board 12g are connected to the negative terminal 12e and the positive terminal, respectively.

The temperature sensor 12a exposed at the surface of the biological information measuring device 12 measures body temperature, and the pressure sensor 12b measures blood pressure. The control unit 12d has a circuit for controlling the timing of collecting or sending data, the timing of power supply, etc. The measured data is transmitted from the information transmitting unit 12c to an external information processing computer or other device with a receiver by radio.

The biological information measuring device 12 which forms the wearable portable device 13 and the battery pack 11 used as the driving power source for the biological information measuring device 12 are both flexible. Thus, the wearable portable device 13 is flexible enough to cause no discomfort even when it is tightly fitted to a living body for a long time. Also, the battery pack 11 uses coin batteries each of which is protected by a housing. Thus, even when the battery pack 11 becomes deformed, the battery components cannot be damaged as badly as when thin batteries are used.

Also, the battery pack 11 can also be used as the driving power source for a transdermal iontophoretic device. At a surface of a transdermal iontophoretic device, a negative terminal and a positive terminal are exposed. The negative terminal exposed at the surface of the transdermal iontophoretic device is connected to the negative electrode of one of the coin batteries embedded in the battery pack, while the positive terminal exposed at the surface of the transdermal iontophoretic device is connected to the positive electrode of one of the coin batteries embedded in the battery pack.
The transdermal iontophoretic device has a cathode chamber for storing an anionic drug and an anode chamber for storing a cationic drug. The cathode chamber and the anode chamber are exposed at the surface of the transdermal iontophoretic device. Also, the negative terminal is connected to the cathode chamber, while the positive terminal is connected to the anode chamber. By fitting the cathode chamber and the anode chamber tightly to a living body and applying a voltage between the cathode chamber and the anode chamber, the drugs can be introduced into the living body.

### [Third Embodiment]

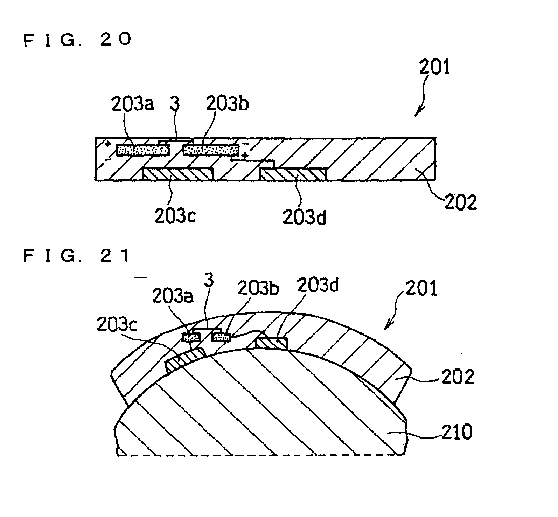

With reference to FIG. 20 and FIG. 21, the third Embodiment describes an electronic device assembly (wearable portable device) including at least one coin battery embedded in an elastic sheet and a device driven by power supplied by the at least one coin battery.

The electronic device assembly is preferably a portable device which operates in contact with the skin of a living body. As such an example, FIG. 20 illustrates a transdermal iontophoretic device 201, which is an electronic device assembly. A transdermal iontophoretic device is a device which utilizes electrical energy to facilitate permeation of an ionic drug through a biological membrane.

The transdermal iontophoretic device 201 of this embodiment includes an elastic sheet 202 and coin batteries 203a and 203b that are embedded in the elastic sheet 202 and connected in series. The transdermal iontophoretic device 201 further includes a cathode chamber 203c for storing an anionic drug and an anode chamber 203d for storing a cationic drug, which are embedded in the elastic sheet 202. The cathode chamber 203c and the anode chamber 203d are exposed at a surface of the elastic sheet 202. The coin battery 203a and the coin battery 203b are connected in series by wiring 3. The negative electrode of the coin battery 203a is electrically connected to the cathode chamber 203c, while the positive electrode of the coin battery 203b is electrically connected to the anode chamber 203d. As illustrated in FIG. 21, the transdermal iontophoretic device 201 is fitted to a living body 210, and a voltage of several volts is applied to the cathode chamber 203c and the anode chamber 203d from the coin batteries 203a and 203b. As a result, the drugs are introduced into the skin of the living body 202. An endogenous ion which makes a pair with one of the drugs is extracted from the skin into the anode chamber 203d. Also, the ions of the anionic drug are exchanged in the cathode chamber 203c. As such, an electrical circuit is established.

The transdermal iontophoretic device 201, which is encapsulated in the elastic sheet, is highly flexible. Thus, when it is tightly fitted to the living body 210, discomfort can be reduced.

Also, the electronic device assembly including at least one coin battery embedded in an elastic sheet and a device driven by power supplied by the at least one coin battery can be a biological information measuring device. In this case, the measuring device 12 described in the second embodiment can be used as the device which is embedded in the elastic sheet together with the at least one coin battery.

### [Fourth Embodiment]

The fourth Embodiment gives a detailed description of modified examples of the battery pack described in the first embodiment.

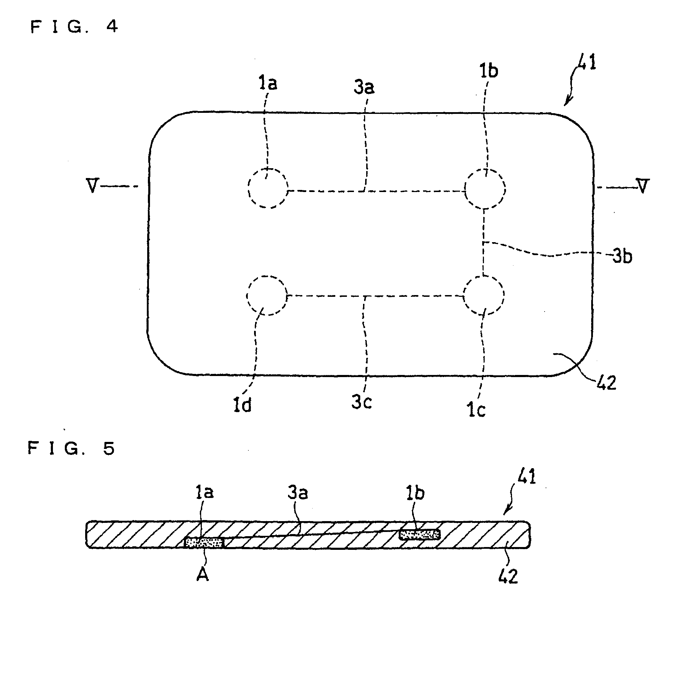

FIG. 4 to FIG. 13 are schematic views of battery packs of this embodiment. In FIG. 4 to FIG. 13, the same constituent components as those of FIG. 1 and FIG. 2 are given the same numbers.

A battery pack 41 of FIG. 4 has a substantially rectangular elastic sheet 42. Coin batteries 1a to 1d are connected by wiring 3.
FIG. 5 is a longitudinal sectional view of the battery pack 41 taken along the line V-V in FIG. 4. In the battery pack 41, the negative electrode A of the coin battery 1a is also exposed at a surface of the elastic sheet 42. Although not shown, the positive electrode B of the coin battery 1d is also exposed at the surface of the elastic sheet 42. The terminals thereof provide connections with the electrode terminals of an electronic device.

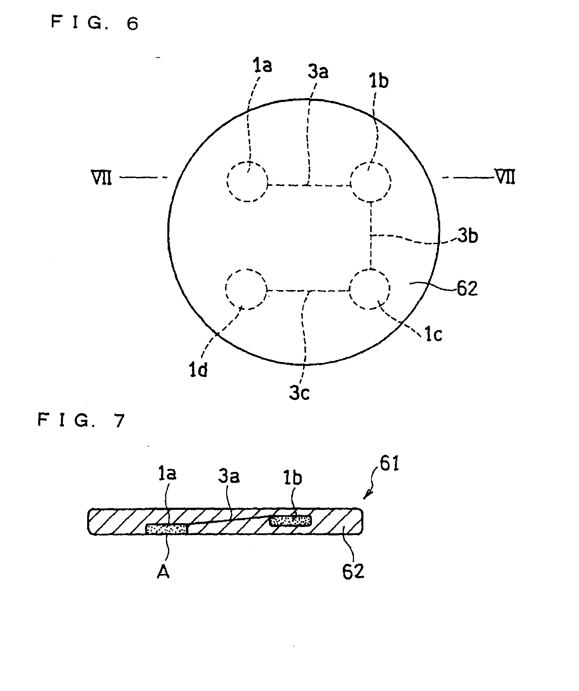

A battery pack 61 of FIG. 6 has a circular elastic sheet 62. Coin batteries 1a to 1d are connected by wiring 3.
FIG. 7 is a longitudinal sectional view of the battery pack 61 taken along the line VII-VII in FIG. 6. In the battery pack 61, the negative electrode A of the coin battery 1a is also exposed at a surface of the elastic sheet 62. Although not shown, the positive electrode B of the coin battery 1d is also exposed at the surface of the elastic sheet 62. The terminals thereof provide connections with the electrode terminals of an electronic device.

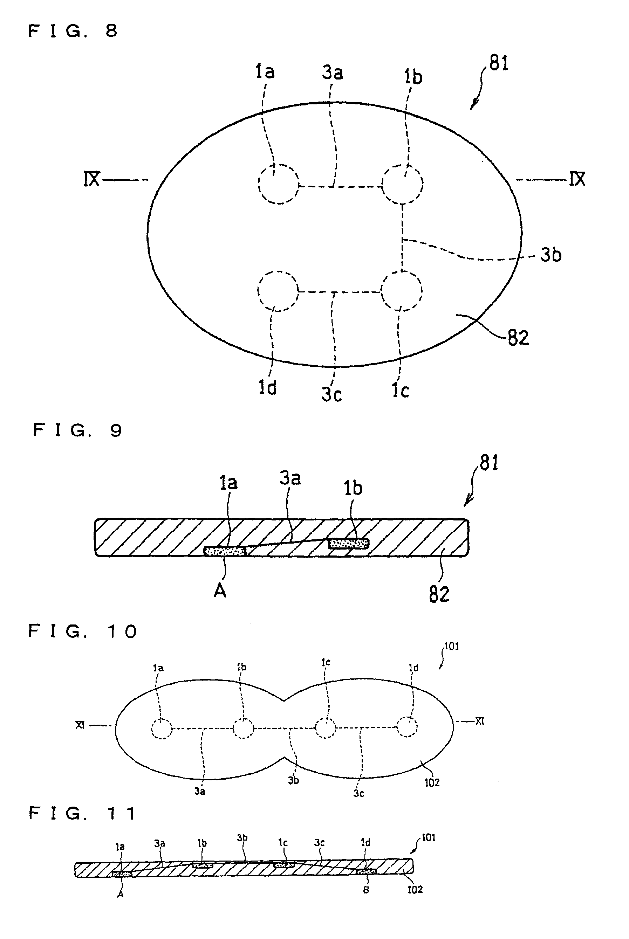

A battery pack 81 of FIG. 8 has an oval elastic sheet 62. Coin batteries 1a to 1d are connected by wiring 3.
FIG. 9 is a longitudinal sectional view of the battery pack 81 taken along the line IX-IX in FIG. 8. In the battery pack 81, the negative electrode A of the coin battery 1a is also exposed at a surface of the elastic sheet 82. Although now shown, the positive electrode B of the coin battery 1d is also exposed at the surface of the elastic sheet 82. The terminals thereof provide connections with the electrode terminals of an electronic device.

In a battery pack 101 of FIG. 10, an elastic sheet 102 has such a shape that two ovals are joined in the directions of the major axes thereof. Coin batteries 1a to 1d are disposed linearly in parallel with the directions of the major axes of the two ovals. The coin batteries 1a to 1d are connected by wiring 3.
FIG. 11 is a longitudinal sectional view of the battery pack 101 taken along the line XI-XI in FIG. 10. As illustrated in FIG. 11, in the battery pack 101, the negative electrode A of the coin battery 1a is also exposed at a surface of the elastic sheet 102, while the positive electrode B of the coin battery 1d is also exposed at the surface of the elastic sheet 102. The terminals thereof provide connections with the electrode terminals of an electronic device.

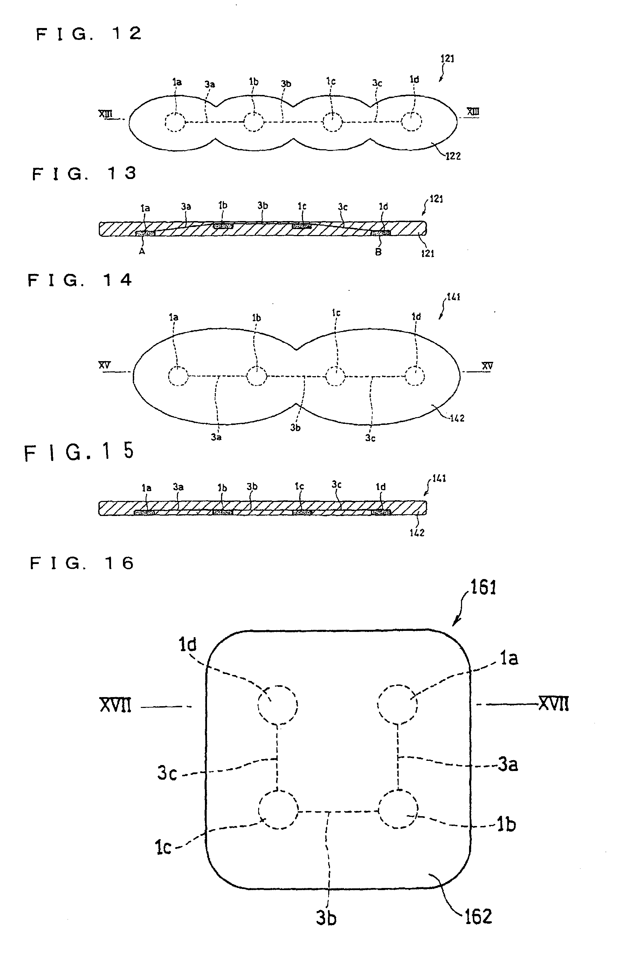

In a battery pack 121 of FIG. 12, an elastic sheet 122 has such a shape that four ovals are joined in the directions of the major axes thereof. Coin batteries 1a to 1d are disposed linearly in parallel with the directions of the major axes of the four ovals. The coin batteries 1a to 1d are connected by wiring 3.
FIG. 13 is a longitudinal sectional view of the battery pack 121 taken along the line XIII-XIII in FIG. 12. As illustrated in FIG. 13, in the battery pack 121, the negative electrode A of the coin battery 1a is also exposed at a surface of the elastic sheet 122, while the positive electrode B of the coin battery 1d is also exposed at the surface of the elastic sheet 122. The terminals thereof provide connections with the electrode terminals of an electronic device.

As described above, by varying the shape of the elastic sheet, battery packs of desired shapes such as polygonal, circular, and oval shapes can be obtained.

In the above description, only two electrodes are exposed at a surface of an elastic sheet, but three or more electrodes can be exposed at a surface of an elastic sheet. Such an example is shown in FIGs. 14 to 15. In FIGs. 14 to 15, the same constituent components as those of FIGs. 1 and 2 are given the same numbers.

A battery pack 141 as illustrated in FIG. 14 basically has the same shape as that of the battery pack 101 illustrated in FIG. 10. However, as illustrated in FIG. 15, the respective terminals of coin batteries 1a to 1d are exposed at a surface of an elastic sheet 142.

As described above, when three or more electrode terminals of three or more batteries are exposed at a surface of the battery pack 141, the battery pack 141 has a plurality of connections with external devices. Thus, various combinations of terminals and external devices are possible. This makes it possible to provide a battery pack capable of providing various voltages or capacities. The number of electrodes exposed at the surface of the elastic sheet 142 can be selected as appropriate, depending on the kind of external devices.

In the above description, the housings of the coin batteries 1 are partially exposed at a surface of an elastic sheet, but connection terminals connected to the batteries 1 may be exposed at a surface of an elastic sheet without exposing the housings of the batteries at a surface of an elastic sheet. Such a structure is described with reference to FIGs. 16 to 19. In FIGs. 16 to 19, the same constituent components as those of FIG. 1 and FIG. 2 are given the same numbers.

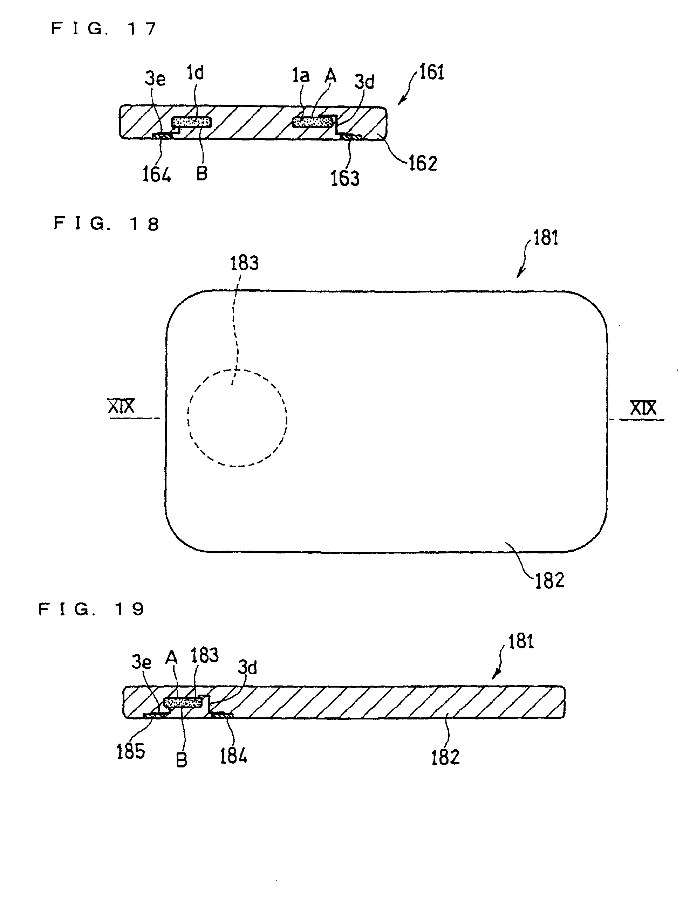

A battery pack 161 of FIG. 16 basically has the same shape as that of the battery pack 11 of FIG. 1. However, as illustrated in FIG. 17, the battery pack 161 has a first connection terminal 163 connected to a coin battery 1a and a second connection terminal 164 connected to a battery 1d. The first connection terminal 163 and the second connection terminal 164 are exposed at different positions on the same plane of the battery pack 161. Also, the first connection terminal 163 and the second connection terminal 164 are connected to the coin battery 1a and the coin battery 1d, respectively, by wiring 3.

The first connection terminal 163 is connected to the negative electrode A of the coin battery 1a, while the second connection terminal 164 is connected to the positive electrode B of the coin battery 1d. That is, the first connection terminal 163 and the second connection terminal 164 have opposite polarities.
The battery pack 161 and an external device can be connected by using the first connection terminal 163 and the second connection terminal 164.

FIG. 18 and FIG. 19 show another example. In a battery pack 181 of FIG. 18 and FIG. 19, a coin battery 183 is embedded in an elastic sheet 182. As illustrated in FIG. 19, the battery pack 181 has a first connection terminal 184 connected to the negative electrode A of the coin battery 183 and a second connection terminal 184 connected to the positive electrode B of the coin battery 183. The first connection terminal 184 and the second connection terminal 185 are exposed at different positions on the same plane of the battery pack 181. Also, the first connection terminal 184 and the second connection terminal 185 are connected to the negative electrode A and the positive electrode B of the coin battery 183, respectively, by wiring 3.

### [Industrial Applicability]

The battery pack of the invention has high flexibility. Thus, the battery pack of the invention can be advantageously used as the power source for devices which are closely fitted to a living body for use for a long time and required to be flexible, such as personal digital assistants, portable electronic appliances, and medical devices. Also, a flexible electronic device assembly can be obtained by incorporating, into the battery pack, a device driven by power supplied by one or more coin batteries contained in the battery pack. Such an electronic device assembly can be used advantageously as a personal digital assistant, a portable electronic appliance, a medical device, etc.

### [Reference Signs List]

1a, 1b, 1c, 1d, 183, 203a, 203b Battery
2, 42, 62, 82, 102, 122, 142, 162, 182, 202 Elastic Sheet
3a, 3b, 3c Wiring
11, 41, 61, 81, 101, 121, 141, 161, 181, 201 Battery Pack
12 External Device
12a First External Device Terminal
163,184 First Connection Terminal
164, 185 Second Connection Terminal

## Claims

1. A battery pack comprising an elastic sheet and at least one coin battery embedded in the elastic sheet.

2. The battery pack of claim 1, wherein a positive electrode of the at least one coin battery or a positive terminal connected to the positive electrode and a negative electrode of the at least one coin battery or a negative terminal connected to the negative electrode are exposed at a surface of the elastic sheet.

3. The battery pack of claim 1, wherein the elastic sheet comprises silicone rubber.

4. The battery pack of claim 1,
wherein the at least one coin battery comprises two or more coin batteries disposed in a horizontal direction of the elastic sheet,
the two or more coin batteries are electrically connected by flexible wiring, and
the wiring is disposed mainly in a central part of the thickness of the elastic sheet.

5. The battery pack of claim 4,
wherein the wiring has slack so that it is longer than the shortest distance between electrodes of the two or more coin batteries, and
when the elastic sheet bends, the wiring can deform together with deformation of the elastic sheet due to disappearance of the slack.

6. The battery pack of claim 5, wherein the slack is contained in a void that is formed in the elastic sheet at a given position along the wiring.

7. The battery pack of claim 1, wherein the at least one coin battery comprises two or more coin batteries connected in series.

8. The battery pack of claim 1, wherein the at least one coin battery comprises two or more coin batteries connected in parallel.

9. An electronic device assembly comprising a laminate of the battery pack of claim 1 and a flexible electronic device, the electronic device being driven by power supplied through positive and negative terminals of the battery pack.

10. An electronic device assembly comprising: the battery pack of claim 1; and a device embedded in the elastic sheet of the battery pack, the device being driven by power supplied by the at least one coin battery of the battery pack.

11. The electronic device assembly of claim 9, which is a portable device that operates in contact with the skin of a living body.

12. The electronic device assembly of claim 11, which is a biological information measuring device or a transdermal iontophoretic device.

13. The electronic device assembly of claim 10, which is a portable device that operates in contact with the skin of a living body.

14. The electronic device assembly of claim 13, which is a biological information measuring device or a transdermal iontophoretic device.
